(19) Europäisches Patentamt
European Patent Office
Office européen des brevets

(11) EP 2 524 969 A1

(12) EUROPEAN PATENT APPLICATION

(43) Date of publication:
21.11.2012 Bulletin 2012/47

(51) Int Cl.:
C12Q 1/68 (2006.01)

(21) Application number: 12003142.2

(22) Date of filing: 02.05.2012

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 19.05.2011 JP 2011112451

(71) Applicant: Sony Corporation
Tokyo 108-0075 (JP)

(72) Inventors:
• Nakamura, Tomohiko
Tokyo 108-0075 (JP)
• Segawa, Yuji
Tokyo 108-0075 (JP)
• Watanabe, Hidetoshi
Tokyo 108-0075 (JP)
• Anaguchi, Takanori
Tokyo 141-0031 (JP)

(74) Representative: Müller - Hoffmann & Partner
Innere Wiener Straße 17
81667 München (DE)

(54) **Method, microchip and mixed reagent for analysis of nucleic acid base sequence**

(57) A technology is provided for achieving high efficiency of nucleic acid amplification reaction and high precision of melting curve analysis when performing melting curve analysis in a nucleic acid amplification process of obtaining an amplification product in which the target nucleic acids are continuously arranged via a loop. A method for analysis of nucleic acid base sequence is provided, and includes: performing amplification reaction of target nucleic acid which is targeted for amplification, in presence of a probe nucleic acid that is capable of hybridizing with the target nucleic acid, to obtain an amplification product in which the target nucleic acids are continuously arranged via a loop site and employing as the probe nucleic acid, a probe nucleic acid having a melting temperature which is lower than the reaction temperature of the amplification reaction; and performing melting curve analysis of the amplification product and the probe nucleic acid.

FIG.1A

**Description**

Background

The present disclosure relates to a method for analysis of nucleic acid base sequence, and a microchip and mixed reagent for analysis of nucleic acid base sequence. More particularly, the present disclosure relates to a method or the like, for performing nucleic acid amplification reaction, followed by performing melting curve analysis, to analyze the base sequence of target nucleic acid which is targeted for amplification.

Summary

A temperature at which double-stranded nucleic acid dissociates (melts) into two single-stranded nucleic acids is referred to as a melting temperature Tm. In melting curve analysis, while the double-stranded nucleic acid is gradually heated up and melts into two single-stranded nucleic acids, a signal which changes with the melting is detected, and a change in signal detection quantity relative to a temperature change is measured. The melting temperature can be determined from a melting curve obtained by plotting the change in signal detection quantity relative to the temperature change. Since the melting temperature reflects the homology between the two single-stranded nucleic acids, it is possible to determine homology between the two single-stranded nucleic acids on the basis of the melting temperature. The melting curve analysis is useful to determine the base sequence homology of target nucleic acids with probe nucleic acids, and is utilized to verify specificity of nucleic acid amplification reaction or to determine genotypes such as single nucleotide polymorphisms (SNPs).

A LAMP (Loop-Mediated Isothermal Amplification) process is a process for performing continuous nucleic acid amplification reaction utilizing strand displacement activity under an isothermal condition by the use of four types of primers for six region of a target nucleic acid. In the LAMP process, a template strand having a complementary sequence on each end, in which a loop site is formed on each end by means of self-annealing of the complementary sequence, is synthesized. In the LAMP process, an amplification product with continuously arranged target nucleic acids targeted for amplification, mediated by this loop, is obtained.

As in the LAMP process, in a nucleic acid amplification process of obtaining an amplification product in which the target nucleic acids targeted for amplification are continuously formed via a loop site, there are some constraints for designing a probe nucleic acid, in the case of performing melting curve analysis between the amplification product and the probe nucleic acid. That is, it is desirable to design a probe nucleic acid so as to hybridize with a loop-formed site which is kept single-stranded in the amplification product, to enable the probe nucleic acid to be double-stranded (hybridized) with the amplification product. For example, Japanese Patent Application Laid-open No. 2009-060838 (hereinafter referred to as the Patent Document 1), describes designing a probe nucleic acid for genotype determination of an amplification product by means of the LAMP process, so that the primer may be annealed with a single-stranded loop site that is specifically observed in LAMP reaction (refer to the paragraph [0015] of the Patent Document 1).

In connection with an embodiment of the present disclosure, Japanese Patent Application Laid-open No. 2003-159100 (hereinafter referred to as the Patent Document 2), describes designing oligonucleotide, which is capable of being annealed specifically with a variation site of a non-targeted base sequence, in order to prevent synthesis of the complementary strand from a non-targeted base sequence, in a gene variation detection method employing the LAMP process (refer to the claim 1 of the Patent Document 2).

As in the LAMP process, in a nucleic acid amplification process of obtaining an amplification product in which the target nucleic acids are continuously arranged via a loop site, it is desirable to design a probe nucleic acid so as to hybridize with a loop-formed site in the case of performing melting curve analysis between the amplification product and the probe nucleic acid. Thus, in the case of determining genotypes such as SNPs by means of melting curve analysis, because a primer must be designed so that SNPs or the like may be located at the loop-formed site, the freedom in primer design is low, and designing itself may be difficult.

In the LAMP process, the fifth and sixth primers, referred to as "loop primers", may be provided for a loop-formed site. By employing the loop primers, it may be possible to increase the primer annealing sites as a starting point of amplification reaction, and reduce amplification reaction time in comparison with a case of employing only four types of primers. However, in the case when a probe nucleic acid for melting curve analysis is designed in the loop-formed site, the loop primers and the probe nucleic acid competitively hybridize to the loop-formed site; and therefore, there is a problem that amplification reaction efficiency decreases significantly. If amplification reaction diminishes, signal detection sensitivity in melting curve analysis also diminishes, and a problem with the analysis precision may occur.

Thus, it is desirable to provide a technology for achieving high efficiency of nucleic acid amplification reaction and high precision of melting curve analysis, when performing the melting curve analysis, in a nucleic acid amplification process of obtaining an amplification product in which the target nucleic acids are continuously arranged via a loop site.

According to an embodiment of the present disclosure, there is provided a method for analysis of nucleic acid base sequence, including: performing amplification reaction of target nucleic acid which is targeted for amplification, in presence of a probe nucleic acid that is capable of hybridizing with the target nucleic acid, to obtain an amplification product in which the target nucleic acids are continuously arranged via a loop site, and employing as the probe nucleic acid, a probe nucleic acid having a melting temperature which is lower than the reaction temperature of the amplification reaction; and performing melting curve analysis of the amplification product and the probe nucleic acid.

In this method for analysis of nucleic acid base sequence, since a melting temperature of a probe nucleic acid is lower than a temperature of amplification reaction, even in the case where amplification reaction is performed in the presence of the probe nucleic acid, the probe nucleic acid does not hybridize with another nucleic acid strand.

A nucleic acid strand the melting temperature of which is lower than the melting temperature of a primer for the amplification reaction may be employed as the probe nucleic acid. This may restrain the probe nucleic acid from competitively hybridizing with the primer for the amplification reaction, or from forming a dimer.

In this method for analysis of nucleic acid base sequence, a nucleic acid strand configured to hybridize with a loop site of the amplification product may be employed as the probe nucleic acid. In this case also, the probe nucleic acid does not competitively hybridize with the primer for the amplification reaction in the loop site.

According to an embodiment of the present disclosure, there is provided a microchip for analysis of nucleic acid base sequence including a reagent for reaction of amplifying a target nucleic acid which is targeted for amplification to obtain an amplification product in which the target nucleic acids are continuously arranged via a loop site, a probe nucleic acid configured to be used for melting curve analysis with the amplification product, having a melting temperature which is lower than the temperature of the reaction, and a reaction region which is configured to retain the reagent for the reaction and the probe nucleic acid within it.

This microchip for analysis of nucleic acid base sequence may have a plurality of the reaction region arranged, and the probe nucleic acids having different base sequences may be retained in the respective reaction regions. One or more of the reaction regions of this microchip may retain, as the probe nucleic acid, a reference probe nucleic acid having a base sequence which is complementary to a conserved sequence region of the target nucleic acid.

Further, according to an embodiment of the present disclosure, there is also provided a mixed reagent for analysis of nucleic acid base sequence, including a reagent for reaction of amplifying a target nucleic acid which is targeted for amplification to obtain an amplification product in which the target nucleic acids are continuously arranged via a loop site, and a probe nucleic acid configured to be used for melting curve analysis with the amplification product, having a melting temperature which is lower than the temperature of the reaction. In this mixed reagent, the reagent for the reaction and the probe nucleic acid are mixed with each other.

A nucleic acid amplification process of obtaining an amplification product in which the target nucleic acids are continuously arranged via a loop site encompasses a wide variety of nucleic acid amplification process employing a DNA polymerase with strand displacement activity, and may include a process such as LAMP process or Smart Amp process, for example.

According to an embodiment of the present disclosure, there is provided a technique for achieving high efficiency of nucleic acid amplification reaction and high precision of melting curve analysis, when performing the melting curve analysis in a nucleic acid amplification process of obtaining an amplification product in which the target nucleic acids are continuously arranged via a loop site.

These and other objects, features and advantages of the present disclosure will become more apparent in light of the following detailed description of best mode embodiments thereof, as illustrated in the accompanying drawings.

Brief Description of Drawings

Figs. 1A and 1B are views for illustrating a structure of a microchip for analysis of nucleic acid base sequence, according to an embodiment of the present disclosure 1A;
Fig. 2 is a view illustrating a design region of a primer employed in the Experimental Examples;
Fig. 3 is a drawing-substitute graph depicting an amplification reaction curve measured in Experimental Example 1; and
Fig. 4 is a drawing-substitute graph depicting a melting curve measured in Experimental Example 2.

Detailed Description of Embodiments

Hereinafter, embodiments of the present disclosure will be described with reference to the drawings. It is to be noted that the embodiment described below shows only one example of typical embodiment of the present disclosure, and therefore should not be considered as limiting the scope of the present disclosure. The description will be given in the following order.

1. Method for analysis of nucleic acid base sequence

    (1) Amplification reaction procedure

    (2) Melting curve analysis procedure

2. Microchip for analysis of nucleic acid base sequence

1. Method for analysis of nucleic acid base sequence

(1) Amplification reaction procedure

A method for analysis of nucleic acid base sequence, according to an embodiment of the present disclosure, includes a procedure (amplification reaction procedure) for performing amplification reaction of target nucleic acid for amplification in the presence of a probe nucleic acid to obtain an amplification product in which the target nucleic acids are continuously arranged via a loop site; and a procedure (melting curve analysis procedure) for performing melting curve analysis of the amplification product and the probe nucleic acid.

In the amplification reaction procedure, amplification reaction is performed for nucleic acids whose base sequence is to be analyzed (as the target nucleic acid). Amplification reaction can be performed by means of a process such as LAMP process or Smart Amp process, or the like. An amplification product is obtained, in which the target nucleic acids are continuously arranged via a loop site, by means of amplification reaction.

In the case where amplification reaction is performed by employing the LAMP process, four types of primers are set for six regions of the target nucleic acid, and are caused to react at a predetermined temperature through the use of strand displacement activity. A target nucleic acid for amplification, primers, DNA polymerase with strand displacement activity, substrates (nucleic acid monomer), and reaction buffering solution (buffer) or the like are mixed, and the mixture is maintained at a predetermined temperature (close to 65.degree.C). By this, the reaction proceeds, and an amplification product with the target nucleic acids continuously arranged via a loop site, will be obtained.

Further, in the LAMP process, as the fifth and sixth primers, primers for loop-formed site can be employed. By employing loop primers, it may be possible to increase the primer annealing sites as a starting point of amplification reaction, and reduce amplification reaction time in comparison with a case of employing only four types of primers.

Amplification reaction is performed in the presence of a probe nucleic acid configured to be used for melting curve analysis with an amplification product. A reaction temperature of the amplification reaction is set to be lower than a melting temperature of primers (four primers and two loop primers in the LAMP process, for example) for amplification reaction. A melting temperature of probe nucleic acid is set to be even lower than the reaction temperature of the amplification reaction.

The melting temperature of probe nucleic acid is set to be lower than the temperature of amplification reaction, whereby in the amplification reaction, a probe nucleic acid does not hybridize with another nucleic acid strand. Thus, it may be unlikely that the probe nucleic acid competitively hybridizes with a primer, or form a dimer, so the probe nucleic acid does not decrease the reaction efficiency of the amplification reaction. In particular, even in the case where a probe nucleic acid is designed in loop-formed site of an amplification product, it may be unlikely that the probe nucleic acid competitively hybridizes with a loop primer, thus making it possible to prevent the decrease in reaction efficiency due to competitive hybridization between the probe nucleic acid and the loop primer.

It is preferable that the melting temperature of a probe nucleic acid be set at 5.degree. to 15.degree.C. lower than the reaction temperature of amplification reaction (about 65.degrees.C.). The melting temperature of the probe nucleic acid can be set by a known approach, and can be set at a desired temperature by appropriately designing base sequence of nucleic acids and the lengths thereof. It is preferable that the melting temperature of the probe nucleic acid be lower than the melting temperature of primers for amplification reaction.

More specifically, since it may be possible to compute the melting temperature of probe nucleic acid by means of the following computation formula, for example, the GC content and the length of the probe nucleic acid can be designed to set the computed value to be 5.degree. to 15.degree.C. lower than the reaction temperature of amplification reaction. Since the melting temperature of nucleic acid may change depending on salt concentration of a reaction buffer solution, further preferably, the GC content and the length of the probe nucleic acid is designed with consideration of a salt concentration condition of the reaction buffer solution as well, so that a desired melting temperature is given.

$$\text{Tm (degree.C.)} = 60.8 + 0.41 \times \text{GC\%}) - (500/n)$$

(GC%): GC content (%) in oligonucleotide
n: Length (mer) of oligonucleotide

(2) Melting curve analysis procedure

In the melting curve analysis procedure, first, a reaction solution subsequent to the amplification reaction procedure is cooled to a temperature under the melting temperature of the probe nucleic acid. When the temperature of the reaction solution becomes lower than the melting temperature of the probe nucleic acid by cooling, the probe nucleic acid hybridizes with an amplification product, and forms a double-stranded nucleic acid. The cooling is typically performed from the reaction temperature of amplification reaction (about 65.degree.C.) to the temperature in the range of from room temperature to 40.degree.C.

Next, after the reaction solution is heated, while the double-stranded nucleic acid is gradually heated up and the double-stranded structure of the probe nucleic acid and the amplification product is melted into two single-stranded nucleic acids, a signal which changes with the melting is detected, and a change in signal detection quantity relative to a temperature change is measured. The heating is typically performed from the temperature subsequent to the cooling (the room temperature to 40.degree.C.) up to a temperature about 90.degree.C.

The signal quantity to be detected can be handled as fluorescence intensity which increases or decreases with the melting of the nucleic acids to single-strands, in the case where a probe nucleic acid which generates (or conversely, quenches) fluorescence due to formation of the double-strand. As the probe nuclear acid which quenches fluorescence due to formation of the double-strand, the one referred to as "QProbe", which is commercially available, may be utilized.

Before performing cooling subsequent to the amplification reaction procedure, heat-denaturation of an amplification product can be performed. The heat-denaturation is typically performed by heating a reaction solution up to a temperature of 90.degree to 100.degree.C., preferably up to approximately 95.degree.C. By this heat-denaturation, a double-stranded part of the amplification product, at which the target nucleic acids are continuously arranged, becomes partially dissociated.

Thus, in the case where a probe nucleic acid is designed at a double-stranded part other than the loop-formed site of the amplification product, the probe nucleic acid can also be hybridized, by being cooled, with the region which is dissociated from the double-stranded part, enabling the melting curve analysis of the double-stranded part.

In the method for analysis of nucleic acid base sequence, according to an embodiment of the present disclosure, the melting temperature of probe nucleic acid is set to be lower than the temperature of amplification reaction. Therefore, even in the case where the probe nucleic acid is designed in loop-formed site of the amplification product, it may be possible to prevent the decrease in amplification reaction efficiency due to competitive hybridization between the probe nucleic acid and the loop primer. In addition, by performing heat-denaturation in the melting curve analysis procedure, even in the case where a probe nucleic acid is designed in a double-stranded part other than the loop-formed site of the amplification product, it may be possible to perform the melting curve analysis. Therefore, with the method for analysis of nucleic-acid base sequence, according to an embodiment of the present disclosure, high analysis precision of melting curve analysis is achieved. Further, in the case of determining genotypes such as SNPs by means of melting curve analysis, high degree of freedom in primer design may be allowed because there is no need for the primer to be designed so that SNPs or the like may be located in the loop-formed site.

In the case of determining genotypes such as SNPs by means of melting curve analysis, it is desirable to design a primer based on a region that does not include a polymorphic (variation) site which is targeted for determination. Conversely, it is desirable to design a probe nucleic acid based on a region including a polymorphic site. In this case, while design regions of primer and probe nuclear acid may partially overlap, it is desirable that these regions be defined so as not to be completely coincident with each other.

2. Microchip for analysis of nucleic acid base sequence

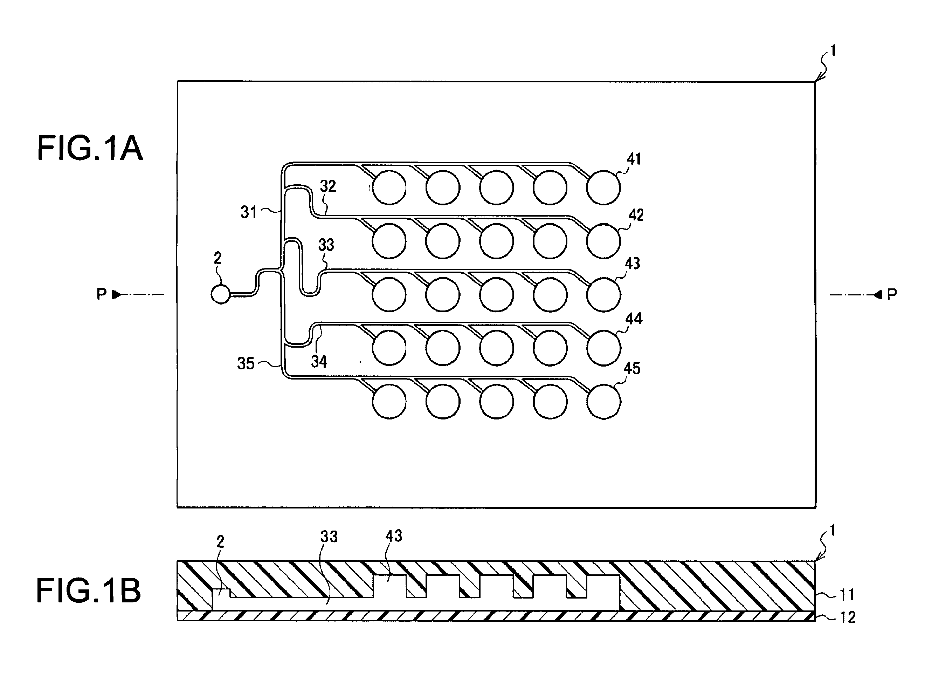

A structure of the microchip for analysis of nucleic acid base sequence, according to an embodiment of the present disclosure (hereinafter, also referred to simply as "microchip") is shown in Figs. 1A and 1B. Fig. 1A shows a top view, and Fig. 1B shows a sectional view corresponding to a cross section taken along the line P-P in Fig. 1A.

On a microchip 1, there are arranged: an introducing portion 2 into which a liquid (sample solution) is to be introduced from the outside; a plurality of wells (reaction regions) 41, 42, 43, 44, and 45 serving as reaction sites for nucleic acid amplification reaction; and flow paths 31, 32, 33, 34, and 35, the each of which communicates with the introducing portion 2 at one end. Herein, by way of example of a case in which a total of 25 wells are arranged at equal intervals in vertical and horizontal matrix of 5 x 5 on the microchip 1, these 25 wells are divided into five sections, and five wells in the same section are designated by same reference numerals. The other end of the flow path 31 is connected to each of the five wells 41 arranged in the same section. Similarly, the other end of each of the flow paths 32, 33, 34, and 35 also is connected to each of the wells 42, 43, 44, and 45 of the respective sets of five wells arranged.

The sample solution introduced from the introducing portion 2 is delivered into the flow paths 31, 32, 33, 34, and 35, and is supplied into the wells 41, 42, 43, 44, and 45. The sample solution may include a target nucleic acid for amplification, the nucleic acid sequence of which is to be analyzed.

The microchip 1 is configured by attaching a substrate layer 12 to a substrate layer 11, forming the introducing portion 2, the flow paths 31, 32, 33, 34, and 35, and the wells 41, 42, 43, 44, and 45. A material for the substrate layers 11 and 12 can be a glass or a variety of plastics (polypropylene, polycarbonate, cyclic olefin polymer, and polydimethylsiloxane). In the case of performing melting curve analysis optically, it is preferable to select the material with optical transparency, less self-fluorescence, and less chromatic dispersion which is with only small optical error, as the material for the substrate layers 11 and 12.

In the wells 41, 42, 43, 44, and 45, a reagent, for reaction of amplifying the target nucleic acid for amplification to obtain an amplification product in which the target nucleic acids for amplification are continuously arranged via a loop site; and a probe nucleic acid, which is configured to be used for melting curve analysis with the amplification product; are retained. A melting temperature of the probe nucleic acid is lower than a temperature of nucleic acid amplification reaction, as described in the method for analysis of nucleic-acid base sequence, according to the present disclosure.

A reagent is a substance required to obtain an amplification product, and specifically, in the case where amplification reaction is performed by employing the LAMP process, for example, the reagent may include a primer (four primers and two loop primers, when required), a DNA polymerase with strand displacement activity, a substrate (nucleic acid monomer), a solute of a reaction buffer solution (buffer), or the like. The mixed reagent for analysis of nucleic acid base sequence, according to an embodiment of the present disclosure, is the mixture in which these reagents and the probe nucleic acid are premixed.

As probe nucleic acids, probe nucleic acids with their different base sequences are retained for the respective sections of the wells 41, 42, 43, 44, and 45. For example, in the case where a known gene variation is to be detected by means of melting curve analysis, a probe nucleic acid for variant type and a probe nucleic acid for wild type are retained in wells as the probe nucleic acids of their different base sequences. The probe nucleic acid for wild type has a base sequence complementary to that of the wild type of the target nucleic acid, the base sequence of which is to be analyzed. The probe nucleic acid for variant type has a base sequence complementary to that of the variant type of the target nucleic acid, the base sequence of which is to be analyzed. Gene variation also includes gene polymorphisms such as SNPs.

After the sample solution including target nucleic acid has been introduced into the well, the amplification reaction procedure and melting curve analysis procedure mentioned above are performed. Then, a melting curve obtained by the well which retained the wild type probe, and a melting curve obtained by the well which retained the variant type probe, are compared with each other. In the case where the target nucleic acid is for a wild type, a melting curve obtained by the well which retained the wild type probe (the base sequence of which is completely coincident with the target nucleic acid for the wild type), indicates higher melting temperature. On the other hand, in the case where the target nucleic acid is for a variant type, a melting curve obtained by the well which retained the variant type probe (the base sequence of which is completely coincident with the target nucleic acid for the variant type), indicates higher melting temperature. In the case where a plurality of variant patterns of gene exists, for example, it may be sufficient for detecting gene variations if a probe nucleic acid for wild type is contained in the well 41, and a number of probe nucleic acids required for variant types corresponding to variant patterns are contained in the wells 42 to 45.

In addition, in the case where an unknown gene variant is to be detected by means of melting curve analysis, for example, a reference probe nucleic acid and detection probe nucleic acids, as the probe nucleic acids of their different base sequences, are retained in the wells. The reference probe nucleic acid has a base sequence complementary to that of the region having the base sequence which is highly conserved, and in which it is evident that no gene variant exists (conserved sequence region). The detection probe nucleic acids each have a base sequence complementary to that of a region, of the target nucleic acid, in which an unknown gene variant may exist.

A result of melting curve analysis depends not only on homology between the base sequences of the two single-strands of nucleic acid, but also on the reaction solution condition, such as salt concentration or coexisting substance concentration, and the concentration of a nucleic acid amplification product. The salt concentration or coexisting substance concentration in reaction solution may change for each sample solution to be analyzed, and the product quantity of the nucleic acid amplification product varies for each reaction. Thus, in the case where an attempt is made to detect an unknown gene variant, since it is uncertain whether the base sequences of a target nucleic acid and a probe nucleic acid are completely coincident or are partially coincident, it may be impossible to easily determine whether a melting curve between the target nucleic acid and the probe nucleic acid is from the wild type or from variant type. In this case, as a probe nucleic acid, a reference probe nucleic acid configured to bond with a region in which it is evident that no gene variant exists, may be retained in advance in the well, and it may be useful to obtain a melting curve in the case where the base sequences are completely coincident. By comparing with the melting curve in the case where the base sequences are completely coincident, it may be possible to determine whether the melting curve obtained by the well in which the detection probe nucleic acid is retained, is from the wild type or from the variant type, easily.

After the sample solution including the target nucleic acid has been introduced into the well, the abovementioned amplification reaction procedure and melting curve analysis procedure are performed. Then, a melting curve obtained by the well which retained the reference probe, and a melting curve obtained by the well which retained the detection probe, are compared with each other. In the case where the target nucleic acid is for the wild type, both the reference probe nucleic acid and the detection probe nucleic acid are completely coincident in base sequence, so the melting curves obtained by the wells which retained these probe nucleic acids (the reference probe and the detection probe), indicate the melting temperatures which are substantially coincident with each other. On the other hand, in the case where the target nucleic acid is for a variant type, a melting curve obtained by the well which retained the reference probe (the base sequence of which is completely coincident with the target nucleic acid for the variant type), indicates a higher melting temperature, and the melting curves obtained by the wells which retained the detection probes indicate lower melting temperatures. In the case where there is a possibility that gene variant patterns exist in more than one site of the target nucleic acid, for example, it may be sufficient for detecting gene variations if the reference probe nucleic acid is contained in the well 41, and a number of detection probe nucleic acids required corresponding to variant patterns are contained in the wells 42 to 45.

The present disclosure may employ the following configurations.

(1) A method for analysis of nucleic acid base sequence, including: performing amplification reaction of target nucleic acid which is targeted for amplification, in presence of a probe nucleic acid that is capable of hybridizing with the target nucleic acid, to obtain an amplification product in which the target nucleic acids are continuously arranged via a loop site, and employing as the probe nucleic acid, a probe nucleic acid having a melting temperature which is lower than the reaction temperature of the amplification reaction; and performing melting curve analysis of the amplification product and the probe nucleic acid.
(2) The method for analysis of nucleic acid base sequence, according to item (1), in which, a nucleic acid strand of which melting temperature is lower than the melting temperature of a primer for the amplification reaction is employed as the probe nucleic acid.
(3) The method for analysis of nucleic acid base sequence, according to item (1) or (2), in which, a nucleic acid strand configured to hybridize with a loop site of the amplification product is employed as the probe nucleic acid.
(4) A microchip for analysis of nucleic acid base sequence, including a reagent for reaction of amplifying a target nucleic acid which is targeted for amplification to obtain an amplification product in which the target nucleic acids are continuously arranged via a loop site, a probe nucleic acid configured to be used for melting curve analysis with the amplification product, having a melting temperature which is lower than the temperature of the reaction, and a reaction region configured to retain the reagent for the reaction and the probe nucleic acid within it.
(5) The microchip for analysis of nucleic acid base sequence, according to item (4), in which a plurality of the reaction regions is arranged, and the probe nucleic acids having different base sequences are retained in the respective reaction regions.
(6) The microchip for analysis of nucleic acid base sequence, according to item (4) or (5), having a reaction region which retains, as the probe nucleic acid, a reference probe nucleic acid having a base sequence which is complementary to a conserved sequence region of the target nucleic acid.
(7) A mixed reagent for analysis of nucleic acid base sequence, including a reagent for reaction of amplifying a target nucleic acid which is targeted for amplification to obtain an amplification product in which the target nucleic acids are continuously arranged via a loop site, and a probe nucleic acid configured to be used for melting curve analysis with the amplification product, having a melting temperature which is lower than the temperature of the reaction, in which the reagent for the reaction and the probe nucleic acid are mixed with each other.

<Experimental Example 1>

1. Nucleic Acid Amplification Reaction

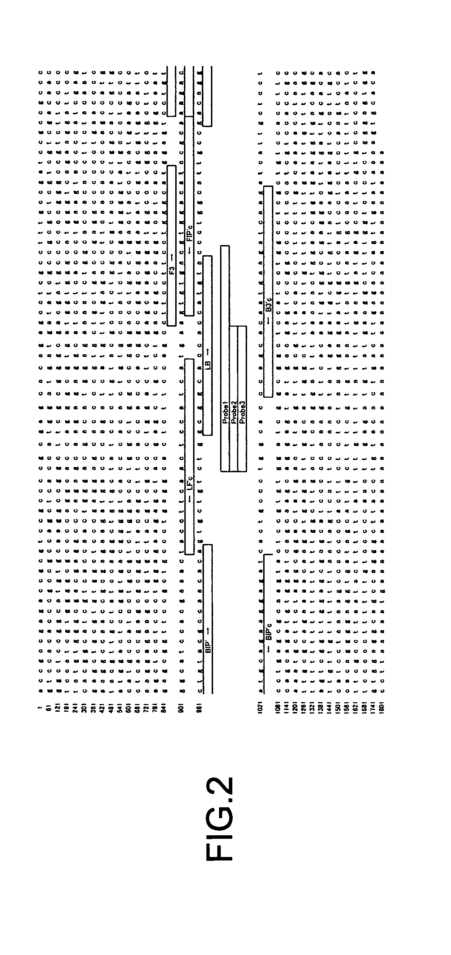

With human beta actin cDNA (refer to the base sequence listed as sequence No.1) as a template nucleic acid strand (as a target for the analyses), nucleic acid amplification reaction was performed by means of the LAMP process. Fig. 2 shows the design regions of the primers, in the base sequence of the template nucleic acid strand. The base sequences (refer to base sequence listed as sequence No.2 to No.7) of the primers, FIP, BIP, LF, LB, F3, and B3 are shown in Table 1.

[Table 1]

| Table.1 | | |
|---|---|---|
| Primer | Base sequence | Sequence number |
| FIP | TGCGGATGTC CACGTCACAC CCTGTGGCAT CCACGAAAC | 2 |
| BIP | AAGACCTGTA CGCCAACACA ATCTCCTTCT GCATCCTGTC | 3 |
| LF | TGATGGAGTT GAAGGTA | 4 |
| LB | GCGGCACCAC CATGTA | 5 |
| F3 | CTTCCTTCCT GGGCAT | 6 |
| B3 | CTTGATCTTC ATTGTGCTGG | 7 |

A probe nucleic acid (Probe 1) with a melting temperature of 62.degree.C. and a probe nucleic acid (Probe 2) with a melting temperature of 50.degree.C. were designed in a region overlapping with that of a loop primer LB. In addition, a probe nucleic acid strand having a base sequence in which a single base mismatches with the base sequence of the template (human beta actin cDNA) was designed as Probe 3. The base sequences of Probe 1 to 3 are shown in Table 2. Each probe was prepared as a custom synthesized QProbe.

[Table 2]

| Table.2 | | |
|---|---|---|
| Probe nucleic acid | Base sequence | Sequence number |
| Probe1 | CTGGCGGCAC CACCATGTAC | 8 |
| Probe2 | CTGGCGGCAC CA | 9 |
| Probe3 | CTGGCGGCAG CA | 10 |

A primer mixture was prepared in accordance with Table 3. By employing a LAMP reagent (available from Eiken Chemical Co., Ltd.), nucleic acid amplification reaction was performed in accordance with Table 4. A reaction temperature was set at 62.degree.C.

[Table 3]

Table.3

| | | |
|---|---|---|
| FIP | (100μM) | 20μL |
| BIP | (100μM) | 20μL |
| LF | (100μM) | 10μL |
| LB | (100μM) | 10μL |
| F3 | (100μM) | 2.5μL |
| B3 | (100μM) | 2.5μL |
| Water | | 35μL |
| Primer mix | | 100μL |

[Table 4]

Table.4

| | |
|---|---|
| Template nucleic acid | 1μL |
| Primer mix | 1μL |
| 2X Buffer | 5μL |
| Probe nucleic acid | 1μL |
| DNA Polymerase | 0.4μL |
| Water | 1.6μL |
| Reaction solution | 10μL |

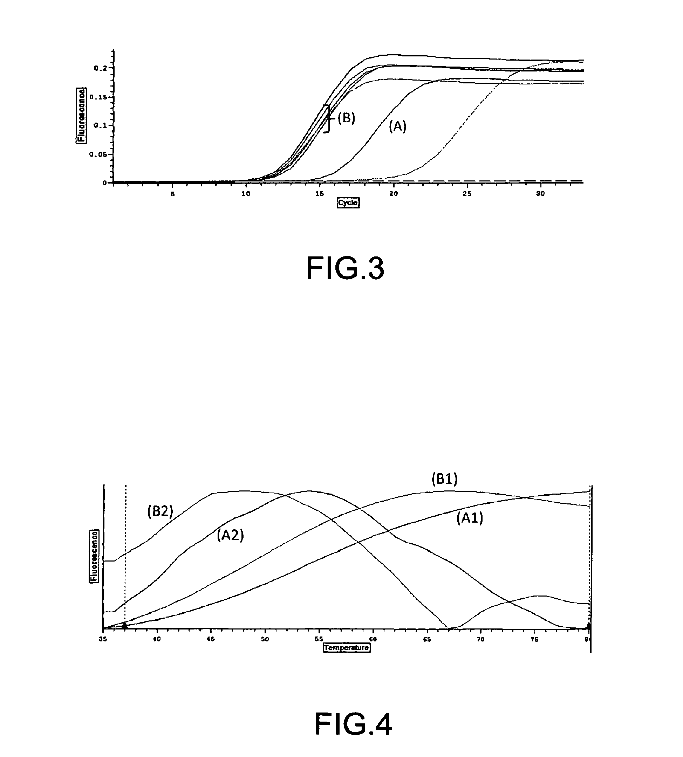

Amplification reaction curves are shown in Fig. 3. In Fig. 3, reference numeral (A) designates an amplification curve obtained by the reaction solution in which Probe 1 whose melting temperature is the same as a reaction temperature was added as a probe nucleic acid. Reference numeral (B) designates an amplification curve obtained by the reaction solution in which Probe 2 whose melting temperature is lower than a reaction temperature was added as a probe nucleic acid.

In the amplification curve (A) of the reaction solution in which Probe 1 was added, it was verified that the rising delayed in comparison with the amplification curve (B) of the reaction solution in which Probe 2 was added, due to Probe 1 with a high melting temperature, which was designed in a region overlapping with that of the loop primer LB, inhibiting the amplification reaction. In contrast, in the reaction solution in which Probe 2 with a low melting temperature was added, it can be said that such lowering of reaction efficiency did not occur or was not likely to occur.

<Experimental Example 2>

2. Melting curve analysis

A result obtained by performing melting curve analysis subsequent to the amplification reaction, is shown in Fig. 4. In Fig. 4, reference numeral (A1) designates a melting curve obtained by the reaction solution in which a probe nucleic acid Probe 2 having a base sequence that is completely coincident with the base sequence of the template (human beta actin cDNA) was added as the probe nucleic acid. Reference numeral (A2) designates a curve indicating a fluorescence intensity increment per unit time, of this melting curve. Reference numeral (B1) designates a melting curve obtained by the reaction solution in which a probe nucleic acid Probe 3 having a base sequence in which a single base mismatches with the base sequence of the template (human beta actin cDNA) was added as the probe nucleic acid. Reference numeral (B2) designates a curve indicating a fluorescence intensity increment per unit time, of this melting curve.

In the reaction solution in which Probe 3 was added, the temperature of the rising in the melting curve was lowered (the melting temperature was lowered) in comparison with the temperature of that of the case when Probe 2 was added. The fluorescence intensity increment (dI/dT) per unit time was 54.0 in the reaction solution in which Probe 2 was added, whereas the increment was 48.0 in the reaction solution in which Probe 3 was added. The above result shows that on the basis of these melting curves, it may be possible to detect the presence of a single-base mismatch between the base sequence in the LAMP amplification product and the base sequence of the probe nucleic acid, and further, it may be possible to detect single nucleotide polymorphisms or other variations which may exist in the target nucleic acid which is targeted for amplification.

Industrial Applicability

According to the embodiments of the present disclosure, when melting curve analysis is performed in a nucleic acid amplification process of obtaining an amplification product in which target nucleic acids are continuously arranged via a loop site, it may be possible to achieve high efficiency of nucleic acid amplification reaction and high precision of melting curve analysis. Therefore, the embodiments of the present disclosure can be suitably used to verify specificity of nucleic acid amplification reaction or to determine genotypes such as single nucleotide polymorphisms (SNPs).

The present disclosure contains subject matter related to that disclosed in Japanese Priority Patent Application JP 2011-112451 filed in the Japan Patent Office on May 19, 2011, the entire content of which is hereby incorporated by reference.

It should be understood by those skilled in the art that various modifications, combinations, sub-combinations and alterations may occur depending on design requirements and other factors insofar as they are within the scope of the appended claims or the equivalents thereof.

**Claims**

1. A method for analysis of nucleic acid base sequence, comprising:

   performing amplification reaction of a target nucleic acid which is targeted for amplification in presence of a probe nucleic acid that is capable of hybridizing with the target nucleic acid, to obtain an amplification product in which the target nucleic acids are continuously arranged via a loop site, and employing as the probe nucleic acid, a probe nucleic acid having a melting temperature which is lower than the reaction temperature of the amplification reaction; and
   performing melting curve analysis of the amplification product and the probe nucleic acid.

2. The method for analysis of nucleic acid base sequence, according to claim 1, wherein
   a nucleic acid strand of which melting temperature is lower than the melting temperature of a primer for the amplification reaction is employed as the probe nucleic acid.

3. The method for analysis of nucleic acid base sequence, according to claim 2, wherein
   a nucleic acid strand configured to hybridize with a loop site of the amplification product is employed as the probe nucleic acid.

4. A microchip for analysis of nucleic acid base sequence, comprising:

   a reagent for reaction of amplifying a target nucleic acid which is targeted for amplification to obtain an amplification product in which the target nucleic acids are continuously arranged via a loop site;
   a probe nucleic acid configured to be used for melting curve analysis with the amplification product, having a melting temperature which is lower than the temperature of the reaction; and
   a reaction region configured to retain the reagent for the reaction and the probe nucleic acid within it.

5. The microchip for analysis of nucleic acid base sequence, according to claim 4, wherein
   a plurality of the reaction regions is arranged, and the probe nucleic acids having different base sequences are retained in the respective reaction regions.

6. The microchip for analysis of nucleic acid base sequence, according to claim 5, having a reaction region which retains, as the probe nucleic acid, a reference probe nucleic acid having a base sequence which is complementary to a conserved sequence region of the target nucleic acid.

7. A mixed reagent for analysis of nucleic acid base sequence, comprising:

   a reagent for reaction of amplifying a target nucleic acid which is targeted for amplification to obtain an amplification product in which the target nucleic acids are continuously arranged via a loop site; and
   a probe nucleic acid configured to be used for melting curve analysis with the amplification product, having a melting temperature which is lower than the temperature of the reaction, wherein
   the reagent for the reaction and the probe nucleic acid are mixed with each other.

EP 2 524 969 A1

FIG.1A

FIG.1B

FIG.2

FIG.3

FIG.4

Europäisches
Patentamt

European
Patent Office

Office européen
des brevets

# EUROPEAN SEARCH REPORT

Application Number

EP 12 00 3142

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YAMAMURA MARIKO ET AL: "Evaluation of a New Rapid Molecular Diagnostic System for Plasmodium falciparum Combined with DNA Filter Paper, Loop-Mediated Isothermal Amplification, and Melting Curve Analysis", JAPANESE JOURNAL OF INFECTIOUS DISEASES, vol. 62, no. 1, January 2009 (2009-01), pages 20-25, XP008156075, ISSN: 1344-6304 * pages 21,22; figures 1-3 * | 1-7 | INV. C12Q1/68 |
| X | UEMURA NATSU ET AL: "Development of a loop-mediated isothermal amplification method for diagnosing Pneumocystis pneumonia.", JOURNAL OF MEDICAL MICROBIOLOGY JAN 2008 LNKD- PUBMED:18065667, vol. 57, no. Pt 1, January 2008 (2008-01), pages 50-57, XP002683356, ISSN: 0022-2615 * the whole document * | 1-7 | |
| X | JP 2009 039106 A (TOYO BOSEKI) 26 February 2009 (2009-02-26) * the whole document * | 1-7 | TECHNICAL FIELDS SEARCHED (IPC) C12Q |
| X | JP 2007 143420 A (TOYO BOSEKI) 14 June 2007 (2007-06-14) * the whole document * | 1-7 | |

-/--

The present search report has been drawn up for all claims

| Place of search | Date of completion of the search | Examiner |
|---|---|---|
| The Hague | 13 September 2012 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 12 00 3142

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VARGA A ET AL: "Use of reverse transcription loop-mediated isothermal amplification for the detection of Plum pox virus", JOURNAL OF VIROLOGICAL METHODS, ELSEVIER BV, NL, vol. 138, no. 1-2, 1 December 2006 (2006-12-01), pages 184-190, XP027892313, ISSN: 0166-0934 [retrieved on 2006-12-01] * the whole document * | 1-7 | |
| A | WO 2010/128206 A1 (EXPRESSION ANALYTICS OY [FI]; ORPANA ARTO [FI]; STENMAN JAKOB [FI]) 11 November 2010 (2010-11-11) * the whole document * | 1-7 | |

**TECHNICAL FIELDS SEARCHED (IPC)**

The present search report has been drawn up for all claims

| Place of search | Date of completion of the search | Examiner |
|---|---|---|
| The Hague | 13 September 2012 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 00 3142

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2012

| Patent document cited in search report | | Publication date | Patent family member(s) | | Publication date |
|---|---|---|---|---|---|
| JP 2009039106 | A | 26-02-2009 | NONE | | |
| JP 2007143420 | A | 14-06-2007 | NONE | | |
| WO 2010128206 | A1 | 11-11-2010 | CN | 102576390 A | 11-07-2012 |
| | | | EP | 2427843 A1 | 14-03-2012 |
| | | | US | 2012101740 A1 | 26-04-2012 |
| | | | WO | 2010128206 A1 | 11-11-2010 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009060838 A
- JP 2003159100 A
- JP 2011112451 A

SEQUENCE LISTING

<110> Sony Corporation

<120> THE METHOD, MICROCHIP AND MIXED REAGENT FOR THE ANALYSIS OF NUCLEIC-ACID BASE SEQUENCE

<130> 318020JP00

<150> JP 2011-112451

<151> 2011-05-19

<160> 10

<170> PatentIn version 3.5

<210> 1

<211> 1852

<212> DNA

<213> Homo sapiens

<400> 1 accgccgaga ccgcgtccgc cccgcgagca cagagcctcg cctttgccga tccgccgccc 60 gtccacaccc gccgccagct caccatggat gatgatatcg ccgcgctcgt cgtcgacaac 120 ggctccggca tgtgcaaggc cggcttcgcg ggcgacgatg cccccc gggc cgtcttcccc 180 tccatcgtgg ggcgccccag gcaccagggc gtgatggtgg gcatgggtca gaaggattcc 240 tatgtgggcg acgaggccca gagcaagaga ggcatcctca ccctgaagta ccccatcgag 300 cacggcatcg tcaccaactg ggacgacatg gagaaaatct ggcaccacac cttctacaat 360 gagctgcgtg tggctcccga ggagcacccc gtgctgctga ccgaggcccc cctgaacccc 420 aaggccaacc gcgagaagat gacccagatc atgtttgaga ccttcaacac cccagccatg 480 tacgttgcta tccaggctgt gctatccctg tacgcctctg ccgtaccac tggcatcgtg 540 atggactccg gtgacggggt cacccacact gtgcccatct acgagggta tgccctcccc 600 catgccatcc tgcgtctgga cctggctggc cgggacctga ctgactacct catgaagatc 660 ctcaccgagc gcggctacag cttcaccacc acggccgagc gggaaatcgt gcgtgacatt 720 aaggagaagc tgtgctacgt cgccctggac ttcgagcaag agatggccac ggctgcttcc 780 agctcctccc tggagaagag ctacgagctg cctgacggcc aggtcatcac cattggcaat 840 gagcggttcc gctgccctga ggcactcttc cagccttcct tcctgggcat ggagtcctgt 900 ggcatccacg aaactacctt caactccatc atgaagtgtg acgtggacat ccgcaaagac 960 ctgtacgcca acacagtgct gtctggcggc accaccatgt accctggcat tgccgacagg 1020 atgcagaagg agatcactgc cctggcaccc

agcacaatga agatcaagat cattgctcct 1080 cctgagcgca agtactccgt

gtggatcggc ggctccatcc tggcctcgct gtccaccttc 1140 cagcagatgt

ggatcagcaa gcaggagtat gacgagtccg gcccctccat cgtccaccgc 1200

aaatgcttct aggcggacta tgacttagtt gcgttacacc ctttcttgac

aaaacctaac 1260 ttgcgcagaa aacaagatga gattggcatg gctttatttg

ttttttttgt tttgttttgg 1320 tttttttttt tttttggct tgactcagga

tttaaaaact ggaacggtga aggtgacagc 1380 agtcggttgg agcgagcatc

ccccaaagtt cacaatgtgg ccgaggactt tgattgcaca 1440 ttgttgtttt

tttaatagtc attccaaata tgagatgcgt tgttacagga agtcccttgc 1500

catcctaaaa gccaccccac ttctctctaa ggagaatggc ccagtcctct

cccaagtcca 1560 cacaggggag gtgatagcat tgctttcgtg taaattatgt

aatgcaaaat ttttttaatc 1620 ttcgccttaa tacttttta ttttgtttta

ttttgaatga tgagccttcg tgccccccct 1680 tcccccttttt ttgtcccccca

acttgagatg tatgaaggct tttggtctcc ctgggagtgg 1740 gtggaggcag

ccagggctta cctgtacact gacttgagac cagttgaata aaagtgcaca 1800

ccttaaaaat gaaaaaaaaa aaaaaaaaaa aaaaaaaaaa aaaaaaaaaa aa 1852

<210> 2 <211> 39 <212> DNA <213> Artificial Sequence <220>

<223> The nucleic acid sequence of the primer FIP. <400> 2

tgcggatgtc cacgtcacac cctgtggcat ccacgaaac 39 <210> 3 <211> 40

<212> DNA <213> Artificial Sequence <220> <223> The nucleic

acid sequence of the primer BIP. <400> 3 aagacctgta cgccaacaca

atctccttct gcatcctgtc 40 <210> 4 <211> 17 <212> DNA <213>

Artificial Sequence <220> <223> The nucleic acid sequence of

the primer LF. <400> 4 tgatggagtt gaaggta 17 <210> 5 <211> 16

<212> DNA <213> Artificial Sequence <220> <223> The nucleic

acid sequence of the primer LB. <400> 5 gcggcaccac catgta 16

<210> 6 <211> 16 <212> DNA <213> Artificial Sequence <220>

<223> The nucleic acid sequence of the primer F3. <400> 6

cttccttcct gggcat 16 <210> 7 <211> 20 <212> DNA <213>

Artificial Sequence <220> <223> The nucleic acid sequence of

the primer B3. <400> 7 cttgatcttc attgtgctgg 20 <210> 8 <211>

20 <212> DNA <213> Artificial Sequence <220> <223> The nucleic

acid sequence of the Probe 1. <400> 8 ctggcggcac caccatgtac 20

<210> 9 <211> 12 <212> DNA <213> Artificial Sequence <220>

```
<223> The nucleic acid sequence of the Probe 2. <400> 9
ctggcggcac ca 12 <210> 10 <211> 12 <212> DNA <213> Artificial
Sequence <220> <223> The nucleic acid sequence of the Probe 3.
<400> 10 ctggcggcag ca 12
```